# EUROPEAN PATENT SPECIFICATION

(11) **EP 2 682 144 B1**
(45) Date of publication and mention of the grant of the patent: **20.09.2017**
(21) Application number: 12752663.0
(22) Date of filing: 14.02.2012
(51) Int. Cl.: A61M 5/32, A61M 5/34, A61M 5/50

(54) **AUTOROTATING RETRACTION STRUCTURE OF NEEDLE SEAT COMPONENTS OF SAFE SYRINGE**
SELBSTROTIERENDE RÜCKZUGSSTRUKTUR FÜR NADELSITZKOMPONENTEN EINER SICHERHEITSSPRITZE
STRUCTURE DE RÉTRACTION PAR AUTOROTATION DE COMPOSANTS DE SIÈGE D'AIGUILLE D'UNE SERINGUE DE SÉCURITÉ

(30) Priority: 02.03.2011 CN 201110050259
(43) Date of publication of application: 08.01.2014
(73) Proprietor: Chang, Shu-Ming, Nantou County 55343 (TW)
(72) Inventor: Chang, Shu-Ming, Nantou County 55343 (TW)
(74) Representative: Lang, Christian
(86) International application number: PCT/CN2012/071139
(87) International publication number: WO 2012/116596

(56) References cited:
- CN-A- 101 898 002
- CN-A- 101 898 002
- CN-A- 101 927 048
- CN-A- 101 927 048
- CN-Y- 2 719 326
- CN-Y- 2 719 326
- US-A- 5 533 970
- US-A- 5 858 000
- US-A- 5 980 487
- US-A- 5 997 511
- US-A- 5 997 511
- US-A1- 2003 212 366
- US-B1- 6 478 779

## Description

### BACKGROUND OF THE INVENTION

### 1. Field of the Invention

The invention relates to an autorotating retraction structure of needle seat components of a safe syringe and more particularly to such a safe syringe wherein after use, a push rod can be broken to leave a portion thereof, a rotating member, and a rotation limiting member in a cylinder so as to prevent the syringe from being used again.

### 2. Description of Related Art

Conventionally, all commercially available syringes are provided with no safety device. They can be used repeatedly. Thus, patients may be subject to contamination when inserting a used syringe into the body for blood transfusion or administering medicine. Further, a medical employee or a cleaner can be pricked by the exposed needle if sufficient care is not taken. Thus, it is desirable among people of the art to provide a safe syringe which cannot be used again after use in order to eliminate above drawback of the conventional syringe.

Conventional retractable safe syringes have a needle seat disposed at a forward opening of a head of cylinder by friction fit. Gap may exist between the needle seat and the cylinder head due to thermal expansion. For example, a quick rise of temperature may form a gap between the needle seat and the cylinder head and in turn it may cause leak in medicine dispensing. Alternatively, a quick decrease of temperature may cause jam, thereby preventing the needle and the needle seat from being retracted into the cylinder after the dispensing. As a result, the whole safe syringe is of no use and discarded.

In response to understanding these problems associated with the typical retraction safe syringes, how to develop a syringe needle seat securely positioned in the cylinder and increase safety of medical employees in use is desired among consumers. Hopefully, it can be developed by relevant industry and it is also the goals of the relevant art.

### SUMMARY OF THE INVENTION

For eliminating defects in the prior art, the invention provides a safe syringe needle retraction structure which significantly improves the needle seat and the cylinder between the appropriate degree of engagement, conveniently allows a retraction movement to a next step, allows an operator to safely one-handed operate, and further increases environmental quality.

To achieve the above object, the invention provides a hypodermic syringe, comprising:
a cylinder including an internal chamber, at least one stopper adjacent to an open end of the chamber, and at least one tracking member disposed at an end of the cylinder, each of the at least one tracking member having a grooved rail facing the chamber;
a needle seat group disposed in a forward end of the cylinder and including a rotating member and a rotation limiting member connected together to form an automatic deflection device wherein the automatic deflection device includes a rotation element in the rotating member, and a non-circular groove in the rotation limiting member, a snapping element is formed and adjacent to the rotation element, at least one L channel is formed on the rotating member for locking or unlocking with the at least one stopper, and a clamping member is formed at a rear portion of the rotating member; and
a push rod including a plurality of ridges on a surface wherein one of the ridges is moveably disposed in the grooved rail so that the push rod is capable of moving along a straight line in the cylinder wherein the push rod further includes a forward manipulation portion and a forward non-circular portion mating with the non-circular groove of the rotation limiting member, and a forward snapping portion secured to the snapping element of the rotating member for retraction.

The rotation limiting member includes a coupling element for joining the rotating member, and the rotating member includes a pivot element for joining the coupling member so as to join the rotating member and the rotation limiting member together.

The rotation limiting member further includes at least one limiting flange on an outer surface facing an open end of the cylinder, each of the at least one limiting flange having a limit rear portion, and the cylinder further includes at least one internal rotation limit element corresponding to the at least one limiting flange respectively.

The push rod further includes an annular flange element disposed on a front portion of the ridges, and the tracking member further includes an annular positioning slot corresponding to the flange element.

The manipulation portion includes a plug, the hypodermic syringe further comprising a connector extending out of a forward end of the ridges, the connector including a hole for receipt of the plug.

Advantages of the invention are as follows: By utilizing the invention, the automatic deflection device engages a groove and the cylinder corresponding to the stopper from the engaging stopper in a locked state and from the open state to an unlocked state, and the push rod can be disposed quickly at a needle seat group and be user-friendly for the next steps of retraction movement of the needle and the needle seat group can be securely positioned in the open end of the cylinder which is uneasy to obtained due to external temperature unpredictable rise such as the temperature soared between the needle and the cylinder, resulting in the injection needle seat jam occurred to cause leak during the retraction, or it is because of the sudden drop in temperature resulting in an excessive engagement, or after the injection it is impossible of retracting the needle and needle seat group into the cylinder, and thus the invention can be achieved by a secure one-handed operation and increase environmental quality.

### BRIEF DESCRIPTION OF THE DRAWINGS

FIG. 1 is an exploded view of a safe syringe of the invention;
FIG. 2 is a rear view of the assembled safe syringe of FIG. 1;
FIG. 3 is a longitudinal sectional view of the rotating member and the rotation limiting member disengaged from the rotating member;
FIG. 4 is a longitudinal sectional view of the rotating member and the rotation limiting member being assembled;
FIG. 5 is a longitudinal sectional view of the safe syringe prior to dispensing liquid medicine;
FIG. 6 is a view similar to FIG. 5 showing the liquid medicine having been dispensed;
FIG. 7 is a longitudinal sectional view of the safe syringe with the need seat group and the needle being disposed in the cylinder and the push rod being broken after use;
FIG. 8 is a longitudinal sectional view showing the push rod being made as two pieces which can be assembled in another configuration; and
FIG. 9 is a longitudinal sectional view of a ring member and a rotation limiting member set to be assembled.

### DETAILED DESCRIPTION OF THE INVENTION

The above and other objects, features and advantages of the invention will become apparent from the following detailed description taken with the accompanying drawings.

### List of reference numerals

needle seat group A; cylinder 10; stopper 11; chamber 12; rotation limiting element 13; finger platform 14; holes 15; push rod 20; ridges 21; breakable portion 22; plastic plug 23; manipulation portion 24; manipulation element 25; inclined groove 26; snapping portion 27; non-circular portion 28; front guide 29; annular flange element 201; rotating member 30; L channel 31; transverse closed end 32; stop element 33; axial open end 34; pivot element 35; sealing ring 36; snapping element 37; rotation element 38; ramp-like guide 39; clamping member 301; rotation limiting member 40; coupling element 41; limiting flange 42; internally threaded projection 43; sealing ring 44; non-circular groove 45; injection member 50; externally threaded section 51; steel needle 52; tracking member 60; grooved rails 61; flange 62; retaining ring groove 63; extending plug 91; connector 92; hole 921.

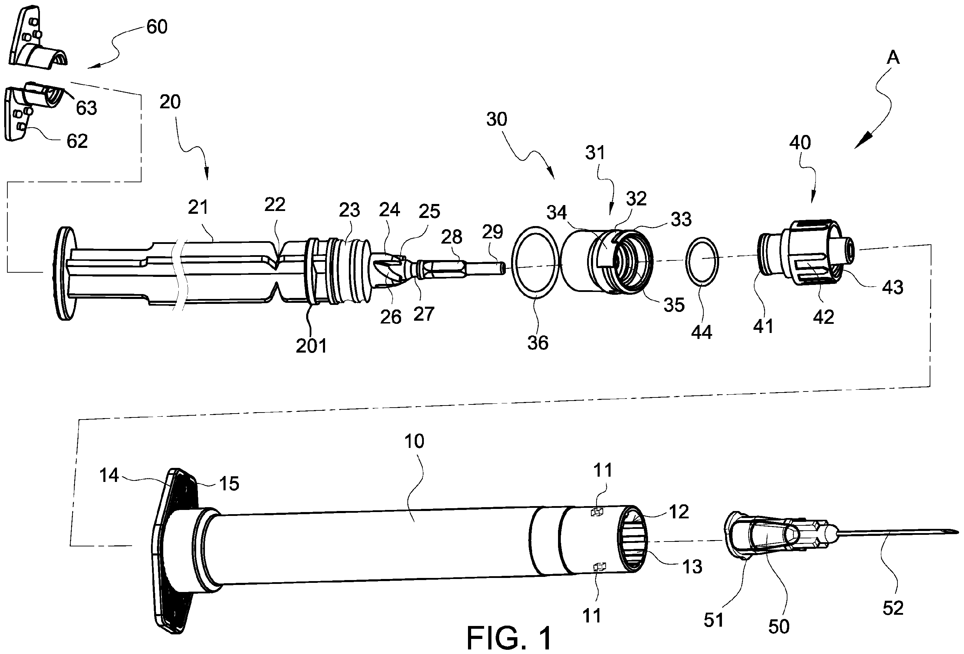

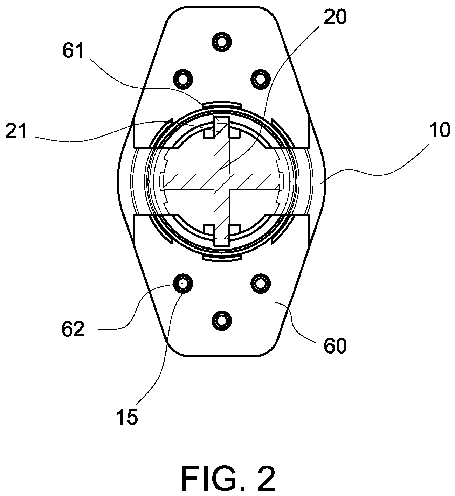

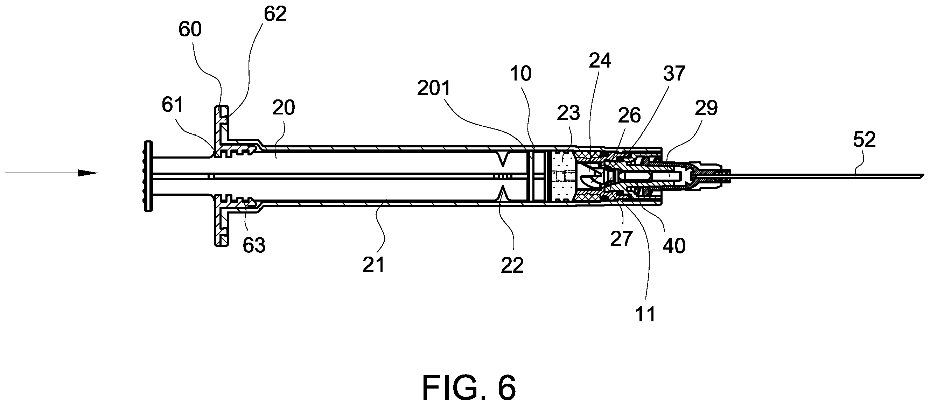

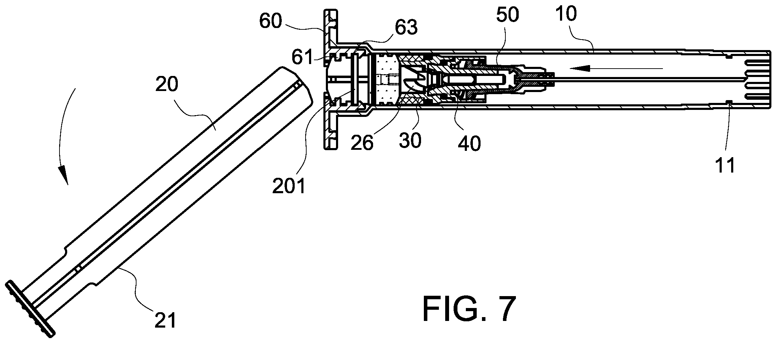

Referring to FIGS. 1 to 7, a hypodermic syringe having a safety mechanism according to a preferred embodiment of the invention comprises the following components:
A cylinder 10 is provided (see FIG. 1). The cylinder 10 is formed with a chamber 12. At an open end of the chamber 12 there is provided at least one stopper 11. Note that there are two stoppers 11 in this embodiment. The stopper 11 is disposed adjacent to a rotation limiting element 13. At least one tracking member 60 is located at a proximal end of the cylinder 10. The proximal end of the cylinder 10 is formed with a laterally extending finger platform 14. The finger platform 14 is provided with a plurality of holes 15 each provided for mounting a flange 62 of the tracking member 60. Within the tracking member 60 facing the chamber 12 there are provided with grooved rails 61. Within the tracking member 60 there is provided a retaining ring groove 63.

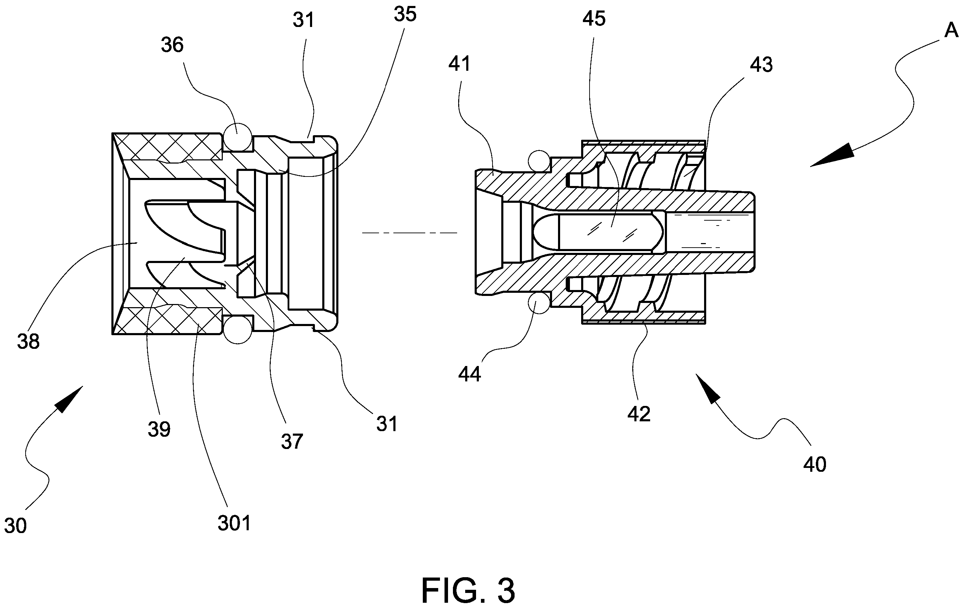

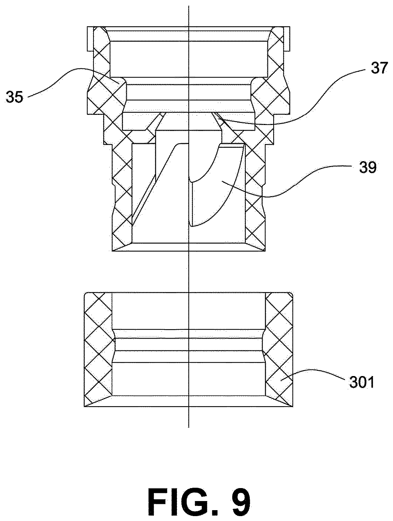

A needle seat group A (see FIG. 1) is disposed in the cylinder 10 and includes a rotating member 30 and a rotation limiting member 40 connected together. A coupling element 41 of the rotation limiting member 40 is formed for joining the rotating member 30. The rotating member 30 has a pivot element 35 adapted to join the coupling member 41 so that the rotating member 30 and the rotation limiting member 40 can be joined together. A sealing ring 44 is provided for a leak proof sealing of the rotation limiting member 40 and the rotating member 30. Referring to FIGS. 3, 4 and 9, a clamping member 301 is formed at a rear portion of the rotating member 30. A sealing ring 36 is provided to fill a gap between the clamping member 301 and the rotating member 30.

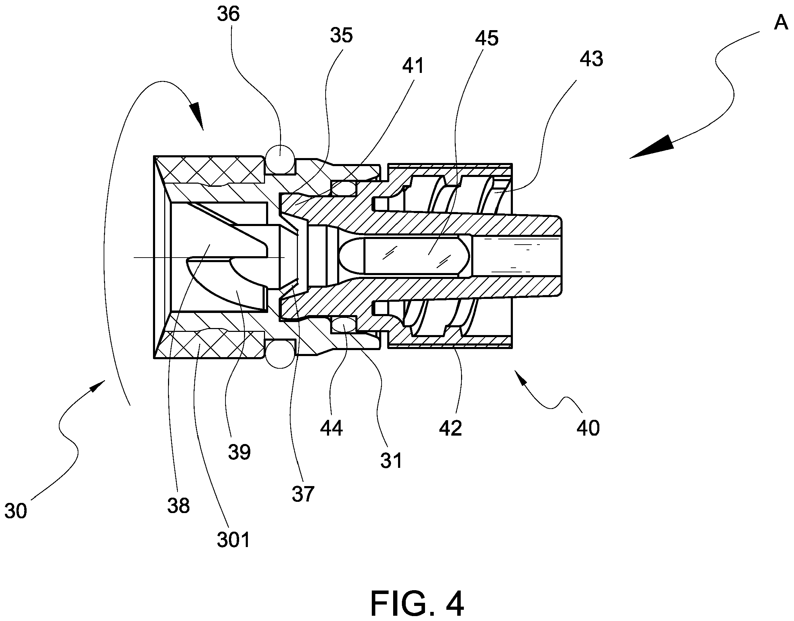

An automatic deflection device is provided between the rotating member 30 and the rotation limiting member 40 (see FIG. 3). The automatic deflection device includes a rotation element 38 in the rotating member 30, and a non-circular groove 45 in the rotation limiting member 40. A ramp-like guide 39 is formed in the rotation element 38. A snapping element 37 is formed and adjacent to the rotation element 38. An L channel 31 is formed on the rotating member 30. The L channel 31 includes a transverse closed end 32, an axial open end 34, and a stop element 33 adjacent to the closed end 32. The rotating member 3 can be turned to dispose the stopper 11 in the L channel 31 in a locked or unlocked position in the cylinder 10. As shown in FIG. 4, the rotation limiting member 40 includes at least one limiting flange 42 on an outer surface facing an open end of the cylinder 10. The cylinder 10 includes at least one internal rotation limit element 13 corresponding to the limiting flange 42. The limiting flange 42 has a limit rear portion. The limiting flanges 42 are disposed between the stoppers 11 when the rotation limiting member 40 is disposed in the chamber 12. Also, the limiting flanges 42 are engaged with the internal rotation limit elements 13. The rotation limiting member 40 includes an internally threaded projection 43 adapted to releasably secure to an externally threaded section 51 at a rear end of an injection member 51. The injection member 50 further comprises a stainless steel needle 52 at a forward end.

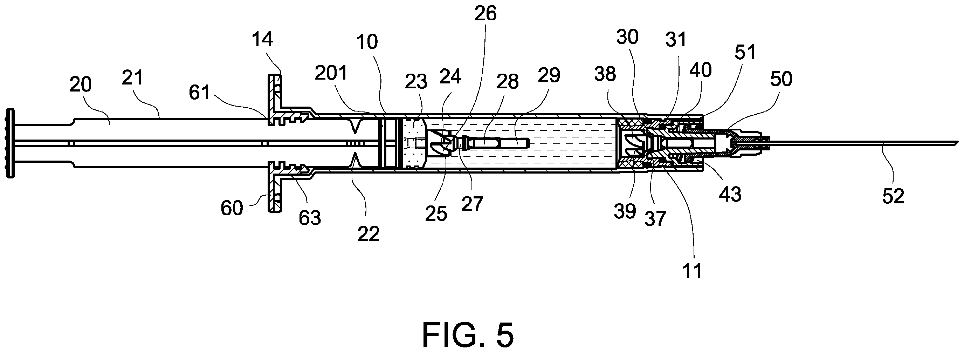

As shown in FIG. 1, a push rod 20, comprises, from a front end to a rear end, a manipulation portion 24, a snapping portion 27, a non-circular portion 28, a front guide 29, a plurality of ridges 21 on a surface, a breakable portion 22 of reduced section, an annular flange element 201 and a plastic plug 23 both disposed on a front portion of the ridges 21. The ridges 21 are moveably disposed in the grooved rails 61 so that the push rod 20 may move along a straight line in the cylinder 10. The push rod 20 can be broken after use by breaking the breakable portion 22. The tracking member 60 has an annular positioning slot 63 corresponding to the flange element 201. As shown in FIG. 5, the non-circular portion 28 of the push rod 20 corresponds to and mates with the non-circular groove 45 of the rotation limiting member 40 so as to assemble so that in cooperation with the ridges 21 disposed in the grooved rails 61 the push rod 20 may move in a straight line. Further, the manipulation portion 24 urges against the rotation element 38 so that the rotating member 30 may deflect automatically. The manipulation portion 24 comprises a manipulation element 25 and an inclined groove 26. The ramp-like guide 39 is adapted to slide on the manipulation element 25 so that the rotating member 30 may deflect automatically when the push rod 20 moves linearly. Thus, the needle seat group A is unlocked in the cylinder 10. Further, the snapping portion 27 may fasten the snapping element 37 to allow a safe retraction of the push rod 20.

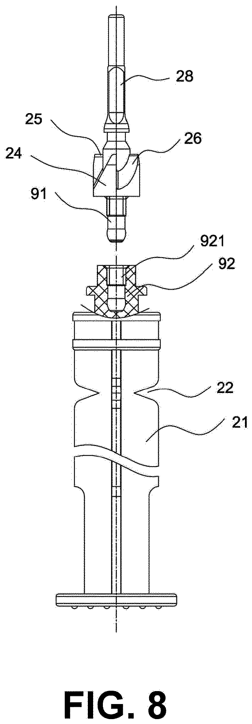

Referring to FIG. 8 specifically, the push rod of the invention can be manufactured separately prior to assembly in another configuration. In detail, the manipulation portion 24 comprises an extending plug 91 adapted to insert into a hole 921 of a connector 92. The manipulation portion 24, the snapping portion 27, the non-circular portion 28, and the front guide 29 consisting the front portion of the push rod 20 can be co-axially arranged with the rear portion of the push rod 20 or not. Preferably, the manipulation portion 24, the snapping portion 27, the non-circular portion 28, and the front guide 29 consisting the front portion of the push rod 20 co-axially arranged with the rear portion of the push rod 20 is more suitable for large CC specifications.

After describing above structure and mechanism, the operation of the invention will now be described as follows:
As shown in FIGS. 5-7, under a normal condition the limiting flanges 42 of the rotation limiting member 40 are disposed in the chamber 12 of the cylinder 10 by urging against the internal rotation limit elements 13. Further, the stop element 33 adjacent to the closed end 32 is locked by to the stopper 11. Thus, the rotation limiting member 40, the rotating member 30, and the cylinder 10 are locked together.

The safety mechanism of the hypodermic syringe of the invention after use including operations such as mixing liquid medicines, injections, and retraction will be discussed in detail below: First, pushes the push rod 20 to an appropriate location, this location being at a location where the snapping portion 27 of the push rod 20 is not locked by the snapping element 37 of the rotating member 30. Next, inserts the needle 52 into a vial and pulls the push rod 20 to create vacuum in the vial so as to draw liquid medicine in the vial into the cylinder 10 until a required quantity is reached. Next, separates the syringe and vial. Injects the liquid medicine into a bottle filled with powder medicine if a mixture with the powder medicine is required. Next, sufficiently mixes them in the bottle. Next, pulls back the needle and replaces it with a new injection member 50 having a smaller needle 42. Next, taps the cylinder 10 and slowly pushes the push rod 20 to propel air and an excess quantity of liquid medicine out of the cylinder 10.

The replacement of the injection member 50 is made possible because, as stated above, the limiting flanges 42 of the rotation limiting member 40 are disposed in the chamber 12 of the cylinder 10 by urging against the internal rotation limit elements 13, and the stop element 33 adjacent to the closed end 32 is locked by to the stopper 11, thereby locking the rotation limiting member 40, the rotating member 30, and the cylinder 10 together.

During the injection, the non-circular portion 28 is secured to the non-circular groove 45 and the ridges 21 are disposed in the grooved rails 61 so that the push rod 20 may move along a straight line in the cylinder 10. The manipulation element 25 is slid on the ramp-like guide 39 to push the rotating member 30 to be disposed in a predetermined angle. Thus, the open end 34 is positioned by the stopper 11 which is in turn not locked by the stop element 33. Thus, the needle seat group A is capable of retracting into the cylinder 10. Further, the snapping portion 27 is pushed to secure to the snapping element 37 so that the needle seat group A can be moved for retraction by the push rod 20. Finally, the needle seat group A and the needle 52 are retracted into the cylinder 10. Also, the flange element 201 is put on the positioning slot 63 for positioning and exposing the breakable portion 22. A medical employee may break the breakable portion 22 to leave a portion thereof, the rotating member 30, and the rotation limiting member 40 in the cylinder 10 so as to prevent the syringe from being used again.

In brief, the hypodermic syringe of the invention employs an automatic rotation retraction structure of a needle seat group, a rotating member, a push rod, a cylinder, and an injection member which cooperate one another to change from a locked position to an unlocked position. After use, the push rod 20 and the needle seat group A can be quickly positioned to facilitate next retracting steps, retracting the needle 52 into the cylinder 10. Advantageously, the needle seat group A can be assembled securely in the cylinder 10 without the adverse effects of the changing ambient temperature such as a loose coupling of the needle seat and the cylinder due to quick temperature change. Further, the invention allows a convenient retraction movement to a next step, allows an operator to safely one-handed operate, and further increases environmental quality.

While the invention has been described in terms of preferred embodiments, those skilled in the art will recognize that the invention can be practiced with modifications within the spirit and scope of the appended claims.

## Claims

1. A hypodermic syringe, comprising:
a cylinder in (10) including an internal chamber, (12) at least one stopper (11) adjacent to an open end of the chamber, and at least one tracking member (60) disposed at an end of the cylinder, each of the at least one tracking member having a grooved rail (61) facing the chamber;
a needle seat group disposed in a forward end of the cylinder and including a rotating member (30) and a rotation limiting member (40) connected together to form an automatic deflection device wherein the automatic deflection device includes a rotation element (38) in the rotating member, and a non-circular groove (45) in the rotation limiting member, a snapping element (37) is formed and adjacent to the rotation element, at least one L channel (31) is formed on the rotating member for locking or unlocking with the at least one stopper, and a clamping member (301) is formed at a rear portion of the rotating member; and
a push rod (20) including a plurality of ridges on a surface wherein one of the ridges is moveably disposed in the grooved rail (62) so that the push rod is capable of moving along a straight line in the cylinder wherein the push rod further includes a forward manipulation portion (24) and a forward non-circular portion (28) mating with the non-circular groove (45) of the rotation limiting member, and a forward snapping portion secured to the snapping element (37) of the rotating member for retraction.

2. The hypodermic syringe of claim 1, wherein the rotation limiting member includes a coupling element for joining the rotating member, and the rotating member includes a pivot element for joining the coupling member so as to join the rotating member and the rotation limiting member together.

3. The hypodermic syringe of claim 1 or 2, wherein the rotation limiting member further includes at least one limiting flange on an outer surface facing an open end of the cylinder, each of the at least one limiting flange having a limit rear portion, and the cylinder further includes at least one internal rotation limit element corresponding to the at least one limiting flange respectively.

4. The hypodermic syringe of claim 1 or 2, wherein the push rod further includes an annular flange element disposed on a front portion of the ridges, and the tracking member further includes an annular positioning slot corresponding to the flange element.

5. The hypodermic syringe of claim 3, wherein the push rod further includes an annular flange element disposed on a front portion of the ridges, and the tracking member further includes an annular positioning slot corresponding to the flange element.

6. The hypodermic syringe of claim 1 or 2, wherein the manipulation portion includes a plug, the hypodermic syringe further comprising a connector extending out of a forward end of the ridges, the connector including a hole for receipt of the plug.

7. The hypodermic syringe of claim 3, wherein the manipulation portion includes a plug, the hypodermic syringe further comprising a connector extending out of a forward end of the ridges, the connector including a hole for receipt of the plug.

8. The hypodermic syringe of claim 4, wherein the manipulation portion includes a plug, the hypodermic syringe further comprising a connector extending out of a forward end of the ridges, the connector including a hole for receipt of the plug.

9. The hypodermic syringe of claim 5, wherein the manipulation portion includes a plug, the hypodermic syringe further comprising a connector extending out of a forward end of the ridges, the connector including a hole for receipt of the plug.

## Patentansprüche

1. Subkutanspritze, umfassend:
einen Zylinder (10), der eine innere Kammer (12), wenigstens einen Anschlag (11), der zu einem offenen Ende der Kammer benachbart ist, und wenigstens ein Nachführteil (60) beinhaltet, das an einem Ende des Zylinders angeordnet ist, wobei jedes des wenigstens einen Nachführteils eine Rillenschiene (61) aufweist, die der Kammer zugewandt ist,
eine Nadelsitzgruppe, die in einem vorderen Ende des Zylinders angeordnet ist und ein Drehteil (30) und ein Rotationsbegrenzungsteil (40) beinhaltet, welche miteinander verbunden sind, um eine automatische Umlenkvorrichtung zu bilden, wobei die automatische Umlenkvorrichtung ein Rotationselement (38) in dem Drehteil und eine nicht-kreisförmige Rille (45) in dem Rotationsbegrenzungsteil beinhaltet, wobei ein Einrastelement (37) benachbart zu dem Rotationselement ausgebildet ist, wobei wenigstens ein L-Kanal (31) an dem Drehteil ausgebildet ist, der mit dem wenigstens einem Anschlag in Eingriff bringbar oder davon lösbar ist, und wobei ein Klemmteil (301) an einem hinteren Abschnitt des Drehteils ausgebildet ist, und
einen Schubstab (20), der an einer Oberfläche eine Vielzahl von Rippen umfasst, wobei eine der Rippen verschiebbar in der Rillenschiene (62) angeordnet ist, sodass der Schubstab entlang einer geraden Linie in dem Zylinder bewegt werden kann, wobei der Schubstab ferner einen vorderen Betätigungsabschnitt (24) und einen vorderen nicht-kreisförmigen Abschnitt (28) umfasst, der mit der nicht-kreisförmigen Rille (45) des Rotationsbegrenzungsteils passend zusammengefügt werden kann, und wobei ein vorderer Einrastabschnitt an dem Einrastelement (37) des Drehteils zum Rückzug befestigt ist.

2. Subkutanspritze nach Anspruch 1, bei welcher das Rotationsbegrenzungsteil ein Kupplungselement zur Verbindung des Drehteils beinhaltet, wobei das Drehteil ein Schwenkelement zur Verbindung mit dem Kupplungselement umfasst, sodass das Drehteil und das Rotationsbegrenzungsteil miteinander kuppelbar sind.

3. Subkutanspritze nach Anspruch 1 oder 2, bei welcher das Rotationsbegrenzungsteil ferner wenigstens einen Begrenzungsflansch an einer einem offenen Ende des Zylinders zugewandten äußeren Oberfläche umfasst, wobei jeder des wenigstens einen Begrenzungsflansches einen hinteren Begrenzungsabschnitt aufweist, und wobei der Zylinder dementsprechend ferner wenigstens ein mit dem wenigstens einen Begrenzungsflansch korrespondierendes innenliegendes Rotationsbegrenzungselement umfasst.

4. Subkutanspritze nach Anspruch 1 oder 2, bei welcher der Schubstab ferner ein ringförmiges Flanschelement umfasst, das an einem Frontabschnitt der Rippen angeordnet ist, wobei das Nachführteil ferner einen mit dem Flanschelement korrespondierenden ringförmigen Positionierschlitz umfasst.

5. Subkutanspritze nach Anspruch 3, bei welcher der Schubstab ferner ein ringförmiges Flanschelement beinhaltet, das an einem Frontabschnitt der Rippen angeordnet ist, wobei das Nachführteil ferner einen mit dem Flanschelement korrespondierenden ringförmigen Positionierschlitz umfasst.

6. Subkutanspritze nach Anspruch 1 oder 2, bei welcher der Betätigungsabschnitt einen Einsatzkörper umfasst, wobei die Subkutanspritze ferner ein Verbindungsstück umfasst, welches sich ausgehend von einem vorderen Ende der Rippen erstreckt, wobei das Verbindungsstück eine Öffnung zur Aufnahme des Einsatzkörpers beinhaltet.

7. Subkutanspritze nach Anspruch 3, bei welcher der Betätigungsabschnitt einen Einsatzkörper umfasst, wobei die Subkutanspritze ferner ein Verbindungsstück umfasst, welches sich ausgehend von einem vorderen Ende der Rippen erstreckt, wobei das Verbindungsstück eine Öffnung zur Aufnahme des Einsatzkörpers beinhaltet.

8. Subkutanspritze nach Anspruch 4, bei welcher der Betätigungsabschnitt einen Einsatzkörper umfasst, wobei die Subkutanspritze ferner ein Verbindungsstück umfasst, welches sich ausgehend von einem vorderen Ende der Rippen erstreckt, wobei das Verbindungsstück eine Öffnung zur Aufnahme des Einsatzkörpers beinhaltet.

9. Subkutanspritze nach Anspruch 5, bei welcher der Betätigungsabschnitt einen Einsatzkörper umfasst, wobei die Subkutanspritze ferner ein Verbindungsstück umfasst, welches sich ausgehend von einem vorderen Ende der Rippen erstreckt, wobei das Verbindungsstück eine Öffnung zur Aufnahme des Einsatzkörpers beinhaltet

## Revendications

1. Seringue hypodermique, comprenant:
un cylindre (10) comportant une chambre interne (12) et au moins une butée (11) adjacente à une extrémité ouverte de la chambre, et au moins un organe de suivi (60) disposé au niveau d'une extrémité du cylindre, chacun de l'au moins un organe de suivi ayant un rail à gorge (61) faisant face à la chambre;
un groupe de sièges d'aiguille disposé dans une extrémité avant du cylindre et comportant un organe rotatif (30) et un organe de limitation de rotation (40) raccordés ensemble pour former un dispositif de fléchissement automatique dans laquelle le dispositif de fléchissement automatique comporte un élément de rotation (38) dans l'organe rotatif, et une gorge non circulaire (45) dans l'organe de limitation de rotation, un élément d'encliquetage (37) est formé et adjacent à l'élément de rotation, au moins un canal en L (31) est formé sur l'organe rotatif pour un verrouillage ou un déverrouillage avec l'au moins une butée, et un organe de serrage (301) est formé au niveau d'une portion arrière de l'organe rotatif; et
une tige de poussée (20) comportant une pluralité d'arêtes sur une surface dans laquelle l'une des arêtes est disposée mobile dans le rail à gorge (62) de sorte que la tige de poussée soit capable de se déplacer le long d'une ligne droite dans le cylindre, dans laquelle la tige de poussée comporte en outre une portion de manipulation vers l'avant (24) et une portion non circulaire vers l'avant (28) s'appariant avec la gorge non circulaire (45) de l'organe de limitation de rotation, et une portion d'encliquetage vers l'avant arrimée à l'élément d'encliquetage (37) de l'organe rotatif pour un retrait.

2. Seringue hypodermique selon la revendication 1, dans laquelle l'organe de limitation de rotation comporte un élément de couplage pour assembler l'organe rotatif, et l'organe rotatif comporte un élément de pivot pour assembler l'organe de couplage de façon à assembler l'organe rotatif et l'organe de limitation de rotation ensemble.

3. Seringue hypodermique selon la revendication 1 ou 2, dans laquelle l'organe de limitation de rotation comporte en outre au moins une bride de limitation sur une surface extérieure faisant face à une extrémité ouverte du cylindre, chacune de l'au moins une bride de limitation ayant une portion arrière de limite, et le cylindre comporte en outre au moins un élément de limite de rotation interne correspondant à l'au moins une bride de limitation respectivement.

4. Seringue hypodermique selon la revendication 1 ou 2, dans laquelle la tige de poussée comporte en outre un élément de bride annulaire disposé sur une portion avant des arêtes, et l'organe de suivi comporte en outre une fente de positionnement annulaire correspondant à l'élément de bride.

5. Seringue hypodermique selon la revendication 3, dans laquelle la tige de poussée comporte en outre un élément de bride annulaire disposé sur une portion avant des arêtes, et l'organe de suivi comporte en outre une fente de positionnement annulaire correspondant à l'élément de bride.

6. Seringue hypodermique selon la revendication 1 ou 2, dans laquelle la portion de manipulation comporte un bouchon, la seringue hypodermique comprenant en outre un raccord s'étendant hors d'une extrémité vers l'avant des arêtes, le raccord comportant un trou pour recevoir le bouchon.

7. Seringue hypodermique selon la revendication 3, dans laquelle la portion de manipulation comporte un bouchon, la seringue hypodermique comprenant en outre un raccord s'étendant hors d'une extrémité vers l'avant des arêtes, le raccord comportant un trou pour recevoir le bouchon.

8. Seringue hypodermique selon la revendication 4, dans laquelle la portion de manipulation comporte un bouchon, la seringue hypodermique comprenant en outre un raccord s'étendant hors d'une extrémité vers l'avant des arêtes, le raccord comportant un trou pour recevoir le bouchon.

9. Seringue hypodermique selon la revendication 5, dans laquelle la portion de manipulation comporte un bouchon, la seringue hypodermique comprenant en outre un raccord s'étendant hors d'une extrémité vers l'avant des arêtes, le raccord comportant un trou pour recevoir le bouchon.
